# EUROPEAN PATENT SPECIFICATION

(11) **EP 1 843 771 B1**
(45) Date of publication and mention of the grant of the patent: **21.09.2011**
(21) Application number: 06706432.9
(22) Date of filing: 26.01.2006
(51) Int. Cl.: A61K 31/506, A61P 9/12

(54) **USE OF PYRIMIDYLAMINOBENZAMIDES FOR THE TREATMENT OF DISEASES THAT RESPOND TO MODULATION OF TIE-2 KINASE ACTIVITY**
VERWENDUNG VON PYRIMIDYLAMINOBENZAMIDEN ZUR BEHANDLUNG VON DURCH MODULATION EINER TIE-2 KINASE-AKTIVITÄT VERURSACHTEN KRANKHEITEN
UTILISATION DE PYRIMIDYLAMINOBENZAMIDES DESTINEES AU TRAITEMENT DE MALADIES QUI REPONDENT A LA MODULATION DE L'ACTIVITE TIE.2 KINASE

(30) Priority: 28.01.2005 US 647895 P; 16.03.2005 US 662404 P
(43) Date of publication of application: 17.10.2007
(73) Proprietor: Novartis AG, 4056 Basel (CH); Novartis Pharma GmbH, 1230 Wien (AT)
(72) Inventor: MANLEY, Paul W., CH-4144 Arlesheim (CH); MARTINY-BARON, Georg, 79336 Herbolzheim (DE); MESTAN, Juergen, 79211 Denzlingen (DE)
(74) Representative: Crawley, Patrick Edward
(86) International application number: PCT/EP2006/000686
(87) International publication number: WO 2006/079539

(56) References cited:
- WO-A-95/09853
- WO-A-2004/005281
- WO-A-2004/029038
- WO-A-2004/091480
- DENNY WILLIAM A: "American Association for Cancer Research - 93rd Annual Meeting. Investigational kinase inhibitors. 6-10 April 2002, San Francisco, CA, USA" IDRUGS : THE INVESTIGATIONAL DRUGS JOURNAL, vol. 5, no. 5, May 2002 (2002-05), pages 416-421, XP002380562
- KIDO M ET AL: "Gene transfer of a TIE2 receptor antagonist prevents pulmonary hypertension in rodents" JOURNAL OF THORACIC AND CARDIOVASCULAR SURGERY, MOSBY-YEAR BOOK, INC., ST. LOUIS, MO, US, vol. 129, no. 2, February 2005 (2005-02), pages 268-276, XP004723943 ISSN: 0022-5223
- CHU D, SULLIVAN C C, DU L, CHO A J, KIDO M, WOLF P L, WEITZMAN M D, JAMIESON S W, THISTLETHWAITE P A: "A new animal model for pulmonary hypertension based on the overexpression of a single gene, angiopoietin-1" THE ANNALS OF THORACIC SURGERY, vol. 77, 2004, pages 449-457, XP002451945
- ARNOLD L D, DIXON R, TALANIAN R, GAZA-BULSECO G, BUMP N, SESHADRI T, BELLAMACINA C, ALLEN K, HOFFKEN W, RIEBEL A, XU Y: "Molecular interactions in crystal structures of potent inhibitors bound to the kinase domain of Tie-2" PROCEEDINGS OF THE AMERICAN ASSOCIATION FOR CANCER RESEARCH, vol. 43, March 2002 (2002-03), page 848, XP001536280

## Description

### Summary of the invention

The present invention relates to the use of pyrimidylaminobenzamide compounds for the preparation of a drug for the treatment of diseases that respond to modulation of kinase activity, especially tie-2 kinase activity, namely for the treatment of pulmonary hypertension.

### Background of the invention

Among the kinases, receptor-type kinases and non-receptor-type kinases can be distinguished, as well as tyrosine and serine/threonine kinases. Among the receptor type tyrosine kinases, Tie-2 (the receptor for angiopoietin-1; also called TEK) is expressed in endothelial cells that line the lumen of blood vessels. Tie-2 has been shown to be involved in endothelial cell migration, sprouting, survival and peri-endothelic cell recruitment during angiogenesis.

Unlike VEGF (vascular endothelial growth factor) and its tyrosine kinase receptors, which control the onset of angiogenesis, angiopoietins and the Tie-2 receptor kinase are involved in vessel stabilization and vascular remodeling. Tie-2 is activated by angiopoietin-1, but antagonized by a second ligand, angiopoietin-2 (ang2). In sites where angiogenesis takes place, the antagonist ang2 is up-regulated. Thus it has not yet been possible to reasonably conclude whether inhibition of Tie-2 promotes or inhibits angiogenesis.

In view of the many mechanisms involved in the pathogenesis of the development and growth of tumors , as well as the pathogenesis of other proliferative diseases, a need exists to find novel and useful modulators of the activity of kinases which frequently play an important role. Thus compounds that modulate Tie-2 activity might affect tumor growth and angiogenesis, this might provide a novel approach to target tumor vessels which are not affected by VEGFR inhibition.

The problem to be solved by the present invention is to provide novel chemical compounds with advantageous properties that are useful in the treatment of pulmonary hypertension.

WO2004/005281 and WO2004/029038 disclose the use of the claimed compounds in the treatment of proliferative diseases, e.g. leukemia, breast cancer, cancer of the colon, lung cancer or cancer of the prostate.

Denny et al. (IDrugs (May 2002), 5 (5), 416 - 421) reports that blocking Tie-2 activity is a promising approach to treat pathologic angiogenesis involved in tumor growth and metastasis.

WO95/09853 shows the use of N-phenyl-2-pyridineamine derivatives for the treatment of tumors.

WO2004/091480 teaches about the use of Tie-2 modulators in the treatment of pulmonary hypertension.

Kido et al. (Journal of Thoracic and Cardiovascular Surgery (2005), 129 (2), 268 - 276) mentions that blocking Tie-2 receptor prevents pulmonary hypertension.

Chu et al. (The Annals of Thoracic Surgery (2004), 43, 449 - 457) reports that inhibition of the expression of Ang-1 may be a useful strategy to prevent and treat pulmonary hypertension.

### General description of the invention

Surprisingly, pyrimidylaminobenzamide compounds of the present invention are capable of inhibiting the activity of Tie-2 kinases and are therefore useful for the treatment of pulmonary hypertension.

### Detailed description of the invention

The present invention relates to the use of pyrimidylaminobenzamide compounds of formula I: 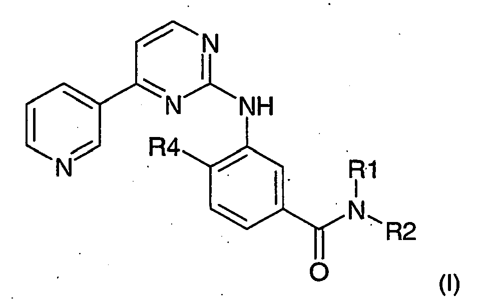 wherein
R₁ represents hydrogen, lower alkyl, lower alkoxy-lower alkyl, acyloxy-lower alkyl, carboxy-lower alkyl, lower alkoxycarbonyl-lower alkyl, or phenyl-lower alkyl;
R₂ represents hydrogen, lower alkyl, optionally substituted by one or more identical or different radicals R₃, cycloalkyl, benzcycloalkyl, heterocyclyl, an aryl group, or a mono- or bicyclic heteroaryl group comprising zero, one, two or three ring nitrogen atoms and zero or one oxygen atom and zero or one sulphur atom, which groups in each case are unsubstituted or mono- or polysubstituted;
and R₃ represents hydroxy, lower alkoxy, acyloxy, carboxy, lower alkoxycarbonyl, carbamoyl, N-mono- or N,N-disubstituted carbamoyl, amino, mono- or disubstituted amino, cycloalkyl, heterocyclyl, an aryl group, or a mono- or bicyclic heteroaryl group comprising zero, one, two or three ring nitrogen atoms and zero or one oxygen atom and zero or one sulphur atom, which groups in each case are unsubstituted or mono- or polysubstituted;
or wherein R₁ and R₂ together represent alkylene with four, five or six carbon atoms optionally mono- or disubstituted by lower alkyl, cycloalkyl, heterocyclyl, phenyl, hydroxy, lower alkoxy, amino, mono- or disubstituted amino, oxo, pyridyl, pyrazinyl or pyrimidinyl; benzalkylene with four or five carbon atoms; oxaalkylene with one oxygen and three or four carbon atoms; or azaalkylene with one nitrogen and three or four carbon atoms wherein nitrogen is unsubstituted or substituted by lower alkyl, phenyl-lower alkyl, lower alkoxycarbonyl-lower alkyl, carboxy-lower alkyl, carbamoyl-lower alkyl, N-mono- or N,N-disubstituted carbamoyl-lower alkyl, cycloalkyl, lower alkoxycarbonyl, carboxy, phenyl, substituted phenyl, pyridinyl, pyrimidinyl, or pyrazinyl;
R₄ represents hydrogen, lower alkyl, or halogen;
and an N-oxide or a pharmaceutically acceptable salt of such a compound for the preparation of a pharmaceutical composition for the treatment of kinase dependent diseases, especially Tie-2 kinase dependent diseases, e.g., as drugs to treat one or more proliferative diseases. The present invention further relates to use of compounds of formula I to treat or prevent kinase dependent diseases, especially Tie-2 dependent diseases.

The general terms used hereinbefore and hereinafter preferably have within the context of this disclosure the following meanings, unless otherwise indicated:
The prefix "lower" denotes a radical having up to and including a maximum of 7, especially up to and including a maximum of 4 carbon atoms, the radicals in question being either linear or branched with single or multiple branching.

Where the plural form is used for compounds, salts, and the like, this is taken to mean also a single compound, salt, or the like.

Any asymmetric carbon atoms may be present in the (R)-, (S)- or (R,S)-configuration, preferably in the (R)- or (S)-configuration. The compounds may thus be present as mixtures of isomers or as pure isomers, preferably as enantiomer-pure diastereomers.

The invention relates also to possible tautomers of the compounds of formula I.

Lower alkyl is preferably alkyl with from and including 1 up to and including 7, preferably from and including 1 to and including 4, and is linear or branched; preferably, lower alkyl is butyl, such as n-butyl, sec-butyl, isobutyl, tert-butyl, propyl, such as n-propyl or isopropyl, ethyl or methyl. Preferably lower alkyl is methyl, propyl or tert-butyl.

Lower acyl is preferably formyl or lower alkylcarbonyl, in particular acetyl.

An aryl group is an aromatic radical which is bound to the molecule via a bond located at an aromatic ring carbon atom of the radical. In a preferred embodiment, aryl is an aromatic radical having 6 to 14 carbon atoms, especially phenyl, naphthyl, tetrahydronaphthyl, fluorenyl or phenanthrenyl, and is unsubstituted or substituted by one or more, preferably up to three, especially one or two substituents, especially selected from amino, mono- or disubstituted amino, halogen, lower alkyl, substituted lower alkyl, lower alkenyl, lower alkynyl, phenyl, hydroxy, etherified or esterified hydroxy, nitro, cyano, carboxy, esterified carboxy, alkanoyl, benzoyl, carbamoyl, N-mono- or N,N-disubstituted carbamoyl, amidino, guanidino, ureido, mercapto, sulfo, lower alkylthio, phenylthio, phenyl-lower alkylthio, lower alkylphenylthio, lower alkylsulfinyl, phenylsulfinyl, phenyl-lower alkylsulfinyl, lower alkylphenylsulfinyl, lower alkylsulfonyl, phenylsulfonyl, phenyl-lower alkylsulfonyl, lower alkylphenylsulfonyl, halogen-lower alkylmercapto, halogen-lower alkylsulfonyl, such as especially trifluoromethanesulfonyl, dihydroxybora (-B(OH)₂), heterocyclyl, a mono- or bicyclic heteroaryl group and lower alkylene dioxy bound at adjacent C-atoms of the ring, such as methylene dioxy. Aryl is more preferably phenyl, naphthyl or tetrahydronaphthyl, 1, which in each case is either unsubstituted or independently substituted by one or two substituents selected from the group comprising halogen, especially fluorine, chlorine, or bromine; hydroxy; hydroxy etherified by lower alkyl, e.g. by methyl, by halogen-lower alkyl, e.g. trifluoromethyl, or by phenyl; lower alkylene dioxy bound to two adjacent C-atoms, e.g. methylenedioxy, lower alkyl, e.g. methyl or propyl; halogen-lower alkyl, e.g. trifluoromethyl; hydroxy-lower alkyl, e.g. hydroxymethyl or 2-hydroxy-2-propyl; lower alkoxy-lower alkyl; e.g. methoxymethyl or 2-methoxyethyl; lower alkoxycarbonyl-lower alkyl, e.g. methoxycarbonylmethyl; lower alkynyl, such as 1-propynyl; esterified carboxy, especially lower alkoxycarbonyl, e.g. methoxycarbonyl, n-propoxy carbonyl or iso-propoxy carbonyl; N-monosubstituted carbamoyl, in particular carbamoyl monosubstituted by lower alkyl, e.g. methyl, n-propyl or iso-propyl; amino; lower alkylamino, e.g. methylamino; di-lower alkylamino, e.g. dimethylamino or diethylamino; lower alkylene-amino, e.g. pyrrolidino or piperidino; lower oxaalkylene-amino, e.g. morpholino, lower azaalkylene-amino, e.g. piperazino, acylamino, e.g. acetylamino or benzoylamino; lower alkylsulfonyl, e.g. methylsulfonyl; sulfamoyl; or phenylsulfonyl.

A cycloalkyl group is preferably cyclopropyl, cyclopentyl, cyclohexyl or cycloheptyl, and may be unsubstituted or substituted by one or more, especially one or two, substitutents selected from the group defined above as substitutents for aryl, most preferably by lower alkyl, such as methyl, lower alkoxy, such as methoxy or ethoxy, or hydroxy, and further by oxo or fused to a benzo ring, such as in benzcyclopentyl or benzcyclohexyl.

Substituted alkyl is alkyl as last defined, especially lower alkyl, preferably methyl; were one or more, especially up to three, substituents may be present, primarily from the group selected from halogen, especially fluorine, amino, N-lower alkylamino, N,N-di-lower alkylamino, N-lower alkanoylamino, hydroxy, cyano, carboxy, lower alkoxycarbonyl, and phenyl-lower alkoxycarbonyl. Trifluoromethyl is especially preferred.

Mono- or disubstituted amino is especially amino substituted by one or two radicals selected independently of one another from lower alkyl, such as methyl; hydroxy-lower alkyl, such as 2-hydroxyethyl; lower alkoxy lower alkyl, such as methoxy ethyl; phenyl-lower alkyl, such as benzyl or 2-phenylethyl; lower alkanoyl, such as acetyl; benzoyl; substituted benzoyl, wherein the phenyl radical is especially substituted by one or more, preferably one or two, substituents selected from nitro, amino, halogen, N-lower alkylamino, N,N-di-lower alkylamino, hydroxy, cyano, carboxy, lower alkoxycarbonyl, lower alkanoyl, and carbamoyl; and phenyl-lower alkoxycarbonyl, wherein the phenyl radical is unsubstituted or especially substituted by one or more, preferably one or two, substituents selected from nitro, amino, halogen, N-lower alkylamino, N,N-di-lower alkylamino, hydroxy, cyano, carboxy, lower alkoxycarbonyl, lower alkanoyl, and carbamoyl; and is preferably N-lower alkylamino, such as N-methylamino, hydroxy-lower alkylamino, such as 2-hydroxyethylamino or 2-hydroxypropyl, lower alkoxy lower alkyl, such as methoxy ethyl, phenyl-lower alkylamino, such as benzylamino, N,N-di-lower alkylamino, N-phenyl-lower alkyl-N-lower alkylamino, N,N-di-lower alkylphenylamino, lower alkanoylamino, such as acetylamino, or a substituent selected from the group comprising benzoylamino and phenyl-lower alkoxycarbonylamino, wherein the phenyl radical in each case is unsubstituted or especially substituted by nitro or amino, or also by halogen, amino, N-lower alkylamino, N,N-di-lower alkylamino, hydroxy, cyano, carboxy, lower alkoxycarbonyl, lower alkanoyl, carbamoyl or aminocarbonylamino. Disubstituted amino is also lower alkylene-amino, e.g. pyrrolidino, 2-oxopyrrolidino or piperidino; lower oxaalkylene-amino, e.g. morpholino, or lower azaalkylene-amino, e.g. piperazino or N-substituted piperazino, such as N-methylpiperazino or N-methoxycarbonylpiperazino.

Halogen is especially fluorine, chlorine, bromine, or iodine, especially fluorine, chlorine, or bromine.

Etherified hydroxy is especially C₈-C₂₀alkyloxy, such as n-decyloxy, lower alkoxy (preferred), such as methoxy, ethoxy, isopropyloxy, or tert-butyloxy, phenyl-lower alkoxy, such as benzyloxy, phenyloxy, halogen-lower alkoxy, such as trifluoromethoxy, 2,2,2-trifluoroethoxy or 1,1,2,2-tetrafluoroethoxy, or lower alkoxy which is substituted by mono- or bicyclic heteroaryl comprising one or two nitrogen atoms, preferably lower alkoxy which is substituted by imidazolyl, such as 1H-imidazol-1-yl, pyrrolyl, benzimidazolyl, such as 1-benzimidazolyl, pyridyl, especially 2-, 3- or 4-pyridyl, pyrimidinyl, especially 2-pyrimidinyl, pyrazinyl, isoquinolinyl, especially 3-isoquinolinyl, quinolinyl, indolyl or thiazolyl.

Esterified hydroxy is especially lower alkanoyloxy, benzoyloxy, lower alkoxycarbonyloxy, such as tert-butoxycarbonyloxy, or phenyl-lower alkoxycarbonyloxy, such as benzyloxycarbonyloxy.

Esterified carboxy is especially lower alkoxycarbonyl, such as tert-butoxycarbonyl, isopropoxycarbonyl, methoxycarbonyl or ethoxycarbonyl, phenyl-lower alkoxycarbonyl, or phenyloxycarbonyl.

Alkanoyl is primarily alkylcarbonyl, especially lower alkanoyl, e.g. acetyl.

N-Mono- or N,N-disubstituted carbamoyl is especially substituted by one or two substituents independently selected from lower alkyl, phenyl-lower alkyl and hydroxy-lower alkyl, or lower alkylene, oxa-lower alkylene or aza-lower alkylene optionally substituted at the terminal nitrogen atom.

A mono- or bicyclic heteroaryl group comprising zero, one, two or three ring nitrogen atoms and zero or one oxygen atom and zero or one sulphur atom, which groups in each case are unsubstituted or mono- or polysubstituted, refers to a heterocyclic moiety that is unsaturated in the ring binding the heteroaryl radical to the rest of the molecule in formula I and is preferably a ring, where in the binding ring, but optionally also in any annealed ring, at least one carbon atom is replaced by a heteroatom selected from the group consisting of nitrogen, oxygen and sulphur; where the binding ring preferably has 5 to 12, more preferably 5 or 6 ring atoms; and which may be unsubstituted or substituted by one or more, especially one or two, substituents selected from the group defined above as substituents for aryl, most preferably by lower alkyl, such as methyl, lower alkoxy, such as methoxy or ethoxy, or hydroxy. Preferably the mono- or bicyclic heteroaryl group is selected from 2H-pyrrolyl, pyrrolyl, imidazolyl, benzimidazolyl, pyrazolyl, indazolyl, purinyl, pyridyl, pyrazinyl, pyrimidinyl, pyridazinyl, 4H-quinolizinyl, isoquinolyl, quinolyl, phthalazinyl, naphthyridinyl, quinoxalyl, quinazolinyl, quinnolinyl, pteridinyl, indolizinyl, 3H-indolyl, indolyl, isoindolyl, oxazolyl, isoxazolyl, thiazolyl, isothiazolyl, triazolyl, tetrazolyl, furazanyl, benzo[d]pyrazolyl, thienyl and furanyl. More preferably the mono- or bicyclic heteroaryl group is selected from the group consisting of pyrrolyl, imidazolyl, such as 1H-imidazol-1-yl, benzimidazolyl, such as 1-benzimidazolyl, indazolyl, especially 5-indazolyl, pyridyl, especially 2-, 3- or 4-pyridyl, pyrimidinyl, especially 2-pyrimidinyl, pyrazinyl, isoquinolinyl, especially 3-isoquinolinyl, quinolinyl, especially 4- or 8-quinolinyl, indolyl, especially 3-indolyl, thiazolyl, benzo[d]pyrazolyl, thienyl, and furanyl. In one preferred embodiment of the invention the pyridyl radical is substituted by hydroxy in ortho position to the nitrogen atom and hence exists at least partially in the form of the corresponding tautomer which is pyridin-(1H)2-one. In another preferred embodiment, the pyrimidinyl radical is substituted by hydroxy both in position 2 and 4 and hence exists in several tautomeric forms, e.g. as pyrimidine-(1H, 3H)2,4-dione.

Heterocyclyl is especially a five, six or seven-membered heterocyclic system with one or two heteroatoms selected from the group comprising nitrogen, oxygen, and sulfur, which may be unsaturated or wholly or partly saturated, and is unsubstituted or substituted especially by lower alkyl, such as methyl, phenyl-lower alkyl, such as benzyl, oxo, or heteroaryl, such as 2-piperazinyl; heterocyclyl is especially 2- or 3-pyrrolidinyl, 2-oxo-5-pyrrolidinyl, piperidinyl, N-benzyl-4-piperidinyl, N-lower alkyl-4-piperidinyl, N-lower alkyl-piperazinyl, morpholinyl, e.g. 2-or 3-morpholinyl, 2-oxo-1H-azepin-3-yl, 2-tetrahydrofuranyl, or 2-methyl-1,3-dioxolan-2-yl.

Salts are especially the pharmaceutically acceptable salts of compounds of formula I.

Such salts are formed, for example, as acid addition salts, preferably with organic or inorganic acids, from compounds of formula I with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

In the presence of negatively charged radicals, such as carboxy or sulfo, salts may also be formed with bases, e.g. metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri(2-hydroxyethyl)amine, or heterocyclic bases, for example N-ethylpiperidine or N,N'-dimethylpiperazine.

When a basic group and an acid group are present in the same molecule, a compound of formula I may also form internal salts.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient.

Compounds within the scope of formula I and the process for their manufacture are disclosed in WO 04/005281 published on January 15, 2004. A preferred compound is 4-Methyl-3-[[4-(3-pyridinyl)-2-pyrimidinyl]amino]-N-[5-(4-methyl-1H-imidazol-1-yl)-3-(trifluoromethyl)phenyl] benzamide.

The terms "treatment" or "therapy" (especially of tyrosine protein kinase dependent diseases or disorders) refer to the prophylactic or preferably therapeutic (including but not limited to palliative, curing, symptom-alleviating, symptom-reducing, kinase-regulating and/or kinase-inhibiting) treatment of said diseases, especially of the diseases mentioned below.

A warm-blooded animal (or patient) is preferably a mammal, especially a human.

Where subsequently or above the term "use" is mentioned (as verb or noun) (relating to the use of a compound of the formula I or a pharmaceutically acceptable salt thereof), this (if not indicated differently or suggested differently by the context) includes any one or more of the following embodiments of the invention, respectively (if not stated otherwise): the use in the treatment of a protein (especially tyrosine, more especially Tie-2) kinase dependent disease, the use for the manufacture of pharmaceutical compositions for use in the treatment of a protein kinase dependent disease, pharmaceutical preparations comprising one or more compounds of the formula I for the treatment of said protein kinase dependent disease, and one or more compounds of the formula I in the treatment of said protein kinase dependent disease, as appropriate and expedient, if not stated otherwise. In particular, diseases to be treated and are thus preferred for "use" of a compound of formula I are selected from (especially tyrosine) protein kinase dependent ("dependent" meaning also "supported", not only "solely dependent") diseases mentioned below, more especially any one or more of these or other diseases that depend on Tie-2, e.g. aberrantly highly-expressed, constitutively activated, normal and/or mutated Tie-2 kinase.

In a preferred sense of the invention, a disease or disorder dependent on activity of a protein (preferably tyrosine) kinase, especially Tie-2, where a compound of the formula I can be used is pulmonary hypertension. The utility of Tie-2 inhibitors for the treatment of pulmonary hypertension is evident from the literature (J Thorac Cardiovasc Surg. 2005 Feb;129(2):268-76; Ann Thorac Surg. 2004 Feb;77(2):449-56; discussion 456-7; Proc Natl Acad Sci U S A. 2003 Oct 14;100(21):12331-6. Epub 2003 Sep 25).

The precise dosage of compound of formula I to be employed for modulating Tie-2 kinase activity depends upon several factors including the host, the nature and the severity of the condition being treated, the mode of administration. The compound of formule I can be administered by any route including orally, parenterally, e.g., intraperitoneally, intravenously, intramuscularly, subcutaneously, intratumorally, or rectally, or enterally. Preferably the compound of formula I is administered orally, preferably at a daily dosage of 1-300 mg/kg body weight or, for most larger primates, a daily dosage of 50-5000, preferably 500-3000 mg. A preferred oral daily dosage is 1-75 mg/kg body weight or, for most larger primates, a daily dosage of 10-2000 mg, administered as a single dose or divided into multiple doses, such as twice daily dosing.

Usually, a small dose is administered initially and the dosage is gradually increased until the optimal dosage for the host under treatment is determined. The upper limit of dosage is that imposed by side effects and can be determined by trial for the host being treated.

Compounds of formula I may be combined with one or more pharmaceutically acceptable carriers and, optionally, one or more other conventional pharmaceutical adjuvants and administered enterally, e.g. orally, in the form of tablets, capsules, caplets, etc. or parenterally, e.g., intraperitoneally or intravenously, in the form of sterile injectable solutions or suspensions. The enteral and parenteral compositions may be prepared by conventional means.

### Examples

The efficacy of pyrimidylaminobenzamide compounds of formula I for the treatment of diseases dependent on Tie-2 kinase activity is illustrated by the results of the following examples.

### Example 1:

### Tie-2 receptor autophosphorylation:

The inhibition of Tie-2 receptor autophosphorylation can be confirmed with an *in vitro* experiment in cells such as transfected COS cells (ATCC Number: CRL-1651), which permanently express human Tie-2 (SwissProt AccNo Q02763), are seeded in complete culture medium (with 10% fetal calf serum = FCS) in 6-well cell-culture plates and incubated at 37°C under 5% CO₂ until they show about 90% confluency. The compounds to be tested are then diluted in culture medium (without FCS, with 0.1 % bovine serum albumin) and added to the cells.

Controls comprise medium without test compounds. After 40min of incubation at 37°C, ortho vanadate is added to give the final concentration of 10 mM. After a further incubation for 20 minutes at 37°C, the cells are washed twice with ice-cold PBS (phosphate-buffered saline) and immediately lysed in 100 µl lysis buffer per well. The lysates are then centrifuged to remove the cell nuclei, and the protein concentrations of the supernatants are determined using a commercial protein assay (BIORAD). The lysates can then either be immediately used or, if necessary, stored at -20°C.

A sandwich ELISA is carried out to measure the Tie-2 phosphorylation: a monoclonal antibody to Tie-2 (for example anti-Tie2 clone AB33, Upstate, Cat Nr. 05-584 or comparable monoclonal antibody) is immobilized using 0.1 ml of a 2µg/ml solution on black ELISA plates (OptiPlate^{™} HTRF-96 from Packard). The plates are then washed and the remaining free protein-binding sites are saturated with 3% TopBlock® (Juro, Cat. # TB232010) in phosphate buffered saline with Tween 20® (polyoxyethylen(20)sorbitane monolaurate, ICI/Uniquema) (PBST). The cell lysates (100 µg protein per well) are then incubated in these plates overnight at 4°C together with an antiphosphotyrosine antibody coupled with alkaline phosphatase (PY20:AP from Zymed). The (plates are washed again and the) binding of the antiphosphotyrosine antibody to the captured phosphorylated receptor is then demonstrated using a luminescent AP substrate (CDP-Star, ready to use, with Emerald II; Applied Biosystems). The luminescence is measured in a Packard Top Count Microplate Scintillation Counter. The difference between the signal of the positive control (stimulated with vanadate) and that of the negative control (not stimulated) corresponds to maximum Tie-2 phosphorylation (= 100 %). The activity of the tested substances is calculated as percent inhibition of maximum Tie-2 phosphorylation, and the concentration of substance that induces half the maximum inhibition is defined as the IC₅₀ (inhibitory dose for 50% inhibition). For compounds of the formula I, preferably IC₅₀ values in the range from 0.0005 to 5 µM can be found, e.g. more preferably from 0.001 to 1 µM. For example, compound 4-Methyl-3-[[4-(3-pyridinyl)-2-pyrimidinyl]amino]-*N*-[5-(4-methyl-1H-imidazol-1-yl)-3-(trifluoromethyl)phenyl]- benzamide, when diluted to 500, 100, 10 or 1 nM shows IC₅₀ values between 10-20 nM.

The results indicate an advantageous selectivity profile of compounds of the formula I with a quite specific inhibition for Tie-2 kinase, where selectivity does not necessarily mean that only Tie-2 kinase is inhibited to an advantageous and pharmaceutically relevant extent.

## Claims

1. Use of a pyrimitiylaminobenzamide of the formula I, or an N-oxide or pharmaceutically acceptable salt thereof, wherein the pyrimidylaminobenzamide of formula I has the following structure: 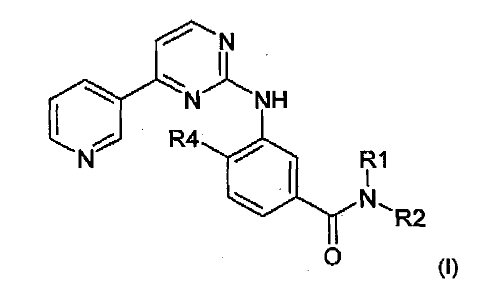 wherein
R₁ represents hydrogen, lower alkyl, lower alkoxy-lower alkyl, acyloxy-lower alkyl, carboxy-lower alkyl, lower alkoxycarbonyl-lower alkyl, or phenyl-lower alkyl;
R₂ represents hydrogen, lower alkyl, optionally substituted by one or more identical or different radicals R₃, cycloalkyl, benzcycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted; and
R₃ represents hydroxy, lower alkoxy, acyloxy, carboxy, lower alkoxycarbonyl, carbamoyl, N-mono- or N,N-disubstituted carbamoyl, amino, mono- or disubstituted amino, cycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted; or wherein
R₁ and R₂ together represent alkylene with four, five or six carbon atoms optionally mono- or disubstituted by lower alkyl, cycloalkyl, heterocyclyl, phenyl, hydroxy, lower alkoxy, amino, mono- or-disubstituted amino, oxo, pyridyl, pyrazinyl or pyrimidinyl; benzalkylene with four or five carbon atoms; oxaalkylene with one oxygen and three or four carbon atoms; or azaalkylene with one nitrogen and three or four carbon atoms wherein nitrogen is unsubstituted or substituted by lower alkyl, phenyl-lower alkyl, lower alkoxycarbonyl-lower alkyl, carboxy-lower alkyl, carbamoyl-lower alkyl, N-mono- or N,N-disubstituted carbamoyl-lower alkyl, cycloalkyl, lower alkoxycarbonyl, carboxy, phenyl, substituted phenyl, pyridinyl, pyrimidinyl, or pyrazinyl;
R₄ represents hydrogen, lower alkyl, or halogen; and
the prefix "lower" denotes a radical having up to and including a maximum of 7 carbon atoms, the radicals in question being either linear or branched with single or multiple branching,
for the manufacture of a pharmaceutical composition for the treatment of a disease that depends on the activity of a protein kinase, which disease is pulmonary hypertension.

2. The use of a pyrimidylaminobenzamide of the formula I according to claim 1 or an N-oxide or pharmaceutically acceptable salt thereof, for the manufacture of a pharmaceutical composition for the treatment of a disease that depends on the activity of the Tie-2 kinase, which disease is pulmonary hypertension.

3. The use according to any one of claims 1 or 2 where the compound of formula I is 4-methyl-3-[[4-(3-pyridinyl)-2-pyrimidinyl]amino]-N-[5-(4-methyl-1H-imidazol-1-yl)-3-(trifluoromethyl)phenyl]-benzamide

4. A pyrimidylaminobenzamide of the formula I, or an N-oxide or pharmaceutically acceptable salt thereof, wherein the pyrimidylaminobenzamide of formula I has the following structure: 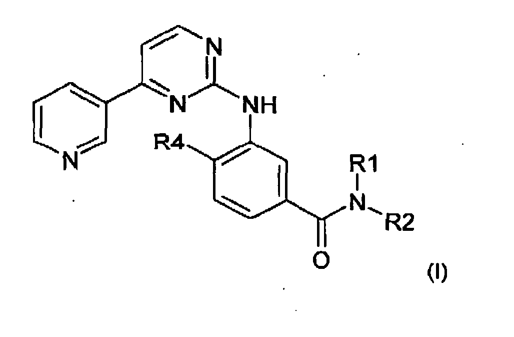 wherein
R₁ represents hydrogen, lower alkyl, lower alkoxy-lower alkyl, acyloxy-lower alkyl, carboxy-lower alkyl, lower alkoxycarbonyl-lower alkyl, or phenyl-lower alkyl;
R₂ represents hydrogen, lower alkyl, optionally substituted by one or more identical or different radicals R₃, cycloalkyl, benzcycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted; and
R₃ represents hydroxy, lower alkoxy, acyloxy, carboxy, lower alkoxycarbonyl, carbamoyl, N-mono- or N,N-disubstituted carbamoyl, amino, mono- or disubstituted amino, cycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted; or wherein
R₁ and R₂ together represent alkylene with four, five or six carbon atoms optionally mono- or disubstituted by lower alkyl, cycloalkyl, heterocyclyl, phenyl, hydroxy, lower alkoxy, amino, mono- or disubstituted amino, oxo, pyridyl, pyrazinyl or pyrimidinyl; benzalkylene with four or five carbon atoms; oxaalkylene with one oxygen and three or four carbon atoms; or azaalkylene with one nitrogen and three or four carbon atoms wherein nitrogen is unsubstituted or substituted by lower alkyl, phenyl-lower alkyl, lower alkoxycarbonyl-lower alkyl, carboxy-lower alkyl, carbamoyl-lower alkyl, N-mono- or N,N-disubstituted carbamoyl-lower alkyl, cycloalkyl, lower alkoxycarbonyl, carboxy, phenyl, substituted phenyl, pyridinyl, pyrimidinyl, or pyrazinyl;
R₄ represents hydrogen, lower alkyl, or halogen; and
the prefix "lower" denotes a radical having up to and including a maximum of 7carbon atoms, the radicals in question being either linear or branched with single or multiple branching,
for use in the treatment of a disease that depends on the activity of a protein kinase, which disease is pulmonary hypertension.

5. The pyrimidylaminobenzamide of the formula I according to claim 4 or an N-oxide or pharmaceutically acceptable salt thereof, for use in the treatment of a disease that depends on the activity of the Tie-2 kinase, which disease is pulmonary hypertension.

6. The pyrimidylaminobenzamide of the formula I according to claim 4 or 5, or an N-oxide or pharmaceutically acceptable salt thereof, for use in the treatment of a disease that depends on the activity of the Tie-2 kinase, wich disease is pulmonary hypertension wherein the compound of formula I is 4-methyl-3-[[4-(3-pyridinyl)-2-pyrimidinyl]amino]-N-[5-(4-methyl-1H-imidazol-1-yl)-3-(trifluoromethyl)phenyl]-benzamide.

## Patentansprüche

1. Verwendung eines Pyrimidylaminobenzamids der Formel 1 oder eines N-Oxids oder pharmazeutisch akzeptablen Salzes hiervon, wobei das Pyrimidylaminobenzamid der Formel I die folgende Struktur besitzt: 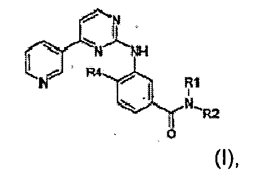 worin
R₁ für Wasserstoff, Niederalkyl, Niederalkoxy-niederalkyl, Acyloxy-niederalkyl, Carboxy-niederalkyl, Niederalkoxycarbonyl-niederalkyl oder Phenyl-niederalkyl steht;
R₂ für Wasserstoff, Niederalkyl, das optional mit einem oder mehreren identischen oder verschiedenen Resten R₃ substituiert ist, Cycloalkyl, Benzocycloalkyl, Heterocyclyl, eine Arylgruppe oder eine mono- oder bicyclische Heteroarylgruppe, die null, ein, zwei oder drei Ringstickstoffatome und null oder ein Sauerstoffatom und null oder ein Schwefelatom umfasst, wobei die Gruppen in jedem Fall unsubstituiert oder mono- oder polysubstituiert sind, steht; und
R₃ für Hydroxy, Niederalkoxy, Acyloxy, Carboxy, Niederalkoxycarbonyl, Carbamoyl, N-mono- oder N,N-disubstituiertes Carbamoyl, Amino, mono- oder disubstituiertes Amino, Cycloalkyl, Heterocyclyl, eine Arylgruppe oder eine mono- oder bicyclische Heteroarylgruppe, die null, ein, zwei oder drei Ringstickstoffatome und null oder ein Sauerstoffatom und null oder ein Schwefelatom umfasst, wobei die Gruppen in jedem Fall unsubstituiert oder mono- oder polysubstituiert sind, steht; oder wobei
R₁ und R₂ zusammen für Alkylen mit vier, fünf oder sechs Kohlenstoffatomen, die optional mono- oder disubstituiert sind mit Niederalkyl, Cycloalkyl, Heterocyclyl, Phenyl, Hydroxy, Niederalkoxy, Amino, mono- oder disubstituiertem Amino, Oxo, Pyridyl, Pyrazinyl oder Pyrimidinyl; Benzalkylen mit vier oder fünf Kohlenstoffatomen; Oxaalkylen mit einem Sauerstoff- und drei oder vier Kohlenstoffatomen; oder Azaalkylen mit einem Stickstoff- und drei- oder vier Kohlenstoffatomen, wobei der Stickstoff unsubstituiert oder mit Niederalkyl, Phenyl-niederalkyl, Niederalkoxycarbonyl-niederalkyl, Carboxy-niederalkyl, Carbamoyl-niederalkyl, N-mono- oder N,N-disubstituiertem Carbamoyl-niederalkyl, Cycloalkyl, Niederalkoxycarbonyl, Carboxy, Phenyl, substituiertem Phenyl, Pyridinyl, Pyrimidinyl, oder Pyrazinyl substituiert ist, stehen;
R₄ für Wasserstoff, Niederalkyl oder Halogen steht; und
das Präfix "Nieder" einen Rest mit bis zu und einschließlich maximal 7 Kohlenstoffatomen bezeichnet, wobei die fraglichen Reste entweder linear oder mit einer einzelnen oder mehreren Verzweigung(en) verzweigt sind,
für die Herstellung einer pharmazeutischen Zusammensetzung für die Behandlung einer Erkrankung, die von der Aktivität einer Proteinkinase abhängt, wobei die Erkrankung pulmonale Hypertonie ist.

2. Verwendung eines Pyrimidylaminobenzamids der Formel I gemäß Anspruch 1 oder eines N-Oxids oder pharmazeutisch akzeptablen Salzes hiervon für die Herstellung einer pharmazeutischen Zusammensetzung für die Behandlung einer Erkrankung, die von der Aktivität der Tie-2-Kinase abhängt, wobei die Erkrankung pulmonale Hypertonie ist.

3. Verwendung gemäß einem der Ansprüche 1 oder 2, worin die Verbindung der Formel I 4-Methyl-3-[[4-(3-pyridinyl)-2-pyrimidinyl]amino]-N-[5-(4-methyl-1H-imidazol-1-yl)-3-(trifluormethyl)phenyl]-benzamid ist.

4. Pyrimidylaminobenzamid der Formel I oder ein N-Oxid oder pharmazeutisch akzeptables Salz hiervon, wobei das Pyrimidylaminobenzamid der Formel I die folgende Struktur besitzt: 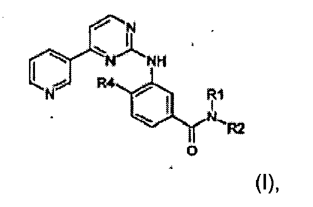 wobei
R₁ für Wasserstoff, Niederalkyl, Niederalkoxy-niederalkyl, Acyloxy-niederalkyl, Carboxy-niederalkyl, Niederalkoxycarbonyl-niederalkyl oder Phenyl-niederalkyl steht;
R₂ für Wasserstoff, Niederalkyl, das optional mit einem oder mehreren identischen oder verschiedenen Resten R₃ substituiert ist, Cycloalkyl, Benzocycloalkyl, Heterocyclyl, eine Arylgruppe oder eine mono- oder bicyclische Heteroarylgruppe, die null, ein, zwei oder drei Ringstickstoffatome und null oder ein Sauerstoffatom und null oder ein Schwefelatom umfasst, wobei die Gruppen in jedem Fall unsubstituiert oder mono- oder polysubstituiert sind; und
R₃ für Hydroxy, Niederalkoxy, Acyloxy, Carboxy, Niederalkoxycarbonyl, Carbamoyl, N-mono- oder N,N,-disubstituiertes Carbamoyl, Amino, mono- oder disubstituiertes Amino, Cycloalkyl, Heterocyclyl, eine Arylgruppe oder eine mono- oder bicyclische Heteroarylgruppe, die null, ein, zwei oder drei Ringstickstoffatome und null oder ein Sauerstoffatom und null oder ein Schwefelatom umfasst, wobei die Gruppen in jedem Fall unsubstituiert oder mono- oder polysubstituiert sind, steht; oder wobei
R₁ und R₂ zusammen für Alkylen mit vier, fünf oder sechs Kohlenstoffatomen, die optional mono- oder disubstituiert sind mit Niederalkyl, Cycloalkyl, Heterocyclyl, Phenyl, Hydroxy, Niederalkoxy, Amino, mono- oder disubstituiertem Amino, Oxo, Pyridyl, Pyrazinyl oder Pyrimidinyl; Benzalkylen mit vier oder fünf Kohlenstoffatomen; Oxaalkylen mit einem Sauerstoff- und drei oder vier Kohlenstoffatomen; oder Azaalkylen mit einem Stickstoff- und drei oder vier Kohlenstoffatomen, wobei der Stickstoff unsubstituiert oder mit Niederalkyl, Phenyl-niederalkyl, Niederalkoxycarbonyl-niederalkyl, Carboxy-niederalkyl, Carbamoyl-niederalkyl, N-mono- oder N,N-disubstituiertem Carbamoyl-niederalkyl, Cycloalkyl, Niederalkoxycarbonyl, Carboxy, Phenyl, substituiertem Phenyl, Pyridinyl, Pyrimidinyl oder Pyrazinyl substituiert ist, stehen;
R₄ für Wasserstoff, Niederalkyl oder Halogen steht; und
das Präfix "Nieder" einen Rest mit bis zu und einschließlich maximal 7 Kohlenstoffatomen bezeichnet, wobei die fraglichen Reste entweder linear oder mit einer einzelnen oder mehreren Verzweigung(en) verzweigt sind,
für die Verwendung bei der Behandlung einer Erkrankung, die von der Aktivität einer Proteinkinase abhängt, wobei die Erkrankung pulmonale Hypertonie ist.

5. Pyrimidylaminobenzamid der Formel I gemäß Anspruch 4 oder ein N-Oxid oder pharmazeutisch akzeptables Salz hiervon zur Verwendung bei der Behandlung einer Erkrankung, die von der Aktivität der Tie-2-Kinase abhängt, wobei die Erkrankung pulmonale Hypertonie ist.

6. Pyrimidylaminobenzamid der Formel I gemäß Anspruch 4 oder 5 oder ein N-Oxid oder pharmazeutisch akzeptables Salz hiervon zur Verwendung bei der Behandlung einer Erkrankung, die von der Aktivität der Tie-2-Kinase abhängt, wobei die Erkrankung pulmonale Hypertonie ist, wobei die Verbindung der Formel I 4-Methyl-3-[[4-(3-pyridinyl)-2-pyrimidinyl]amino]-N-[5-(4-methyl-1H-imidazol-1-yl)-3-(trifluormethyl)phenyl]benzamid ist.

## Revendications

1. Utilisation d'un pyrimidylaminobenzamide de formule I, ou d'un N-oxyde ou sel de celui-ci acceptable sur le plan pharmaceutique, dans laquelle le pyrimidylaminobenzamide de formule I présente la structure suivante : 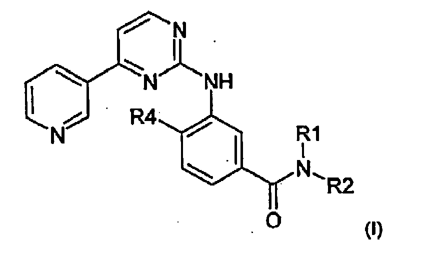 dans laquelle
R₁ représente l'hydrogène, un groupe alkyle inférieur, alcoxy inférieur-alkyle inférieur, acyloxy-alkyle inférieur, carboxy-alkyle inférieur, (alcoxy inférieur)carbonyl-alkyle inférieur ou phényl- alkyle inférieur ;
R₂ représente l'hydrogène, un groupe alkyle inférieur, éventuellement substitué par un ou plusieurs radicaux R₃ identiques ou différents, cycloalkyle, benzocycloalkyle, hétérocyclyle, un groupe aryle, ou un groupe hétéroaryle mono- ou bicyclique comprenant zéro, un, deux ou trois atomes d'azote cyclique et zéro ou un atome d'oxygène et zéro ou un atome de soufre, lesdits groupes étant dans chaque cas non substitués ou mono- ou polysubstitués ; et
R₃ représente un groupe hydroxy, alcoxy inférieur, acyloxy, carboxy, (alcoxy inférieur)carbonyle, carbamoyle, carbamoyle N- monosubstitué ou N,N-disubstitué, amino, amino mono- ou disubstitué, cycloalkyle, hétérocyclyle, un groupe aryle, ou un groupe hétéroaryle mono- ou bicyclique comprenant zéro, un, deux ou trois atomes d'azote cyclique et zéro ou un atome d'oxygène et zéro ou un atome de soufre, lesdits groupes étant dans chaque cas non substitués ou mono- ou polysubstitués ; ou dans laquelle
R₁ et R₂ conjointement représentent un groupe alkylène comportant quatre, cinq ou six atomes de carbone, éventuellement mono- ou disubstitué par un groupe alkyle inférieur, cycloalkyle, hétérocyclyle, phényle, hydroxy, alcoxy inférieur, amino, amino mono- ou disubstitué, oxo, pyridyle, pyrazinyle ou pyrimidinyle ; benzalkylène comportant quatre ou cinq atomes de carbone ; oxoalkylène comportant un oxygène et trois ou quatre atomes de carbone ; ou azaalkylène comportant un atome d'azote et trois ou quatre atomes de carbone, où l'atome d'azote est non substitué ou substitué par un groupe alkyle inférieur, phényl-alkyle inférieur, (alcoxy inférieur)carbonyl-alkyle inférieur, carboxy-alkyle inférieur, carbamoyl-alkyle inférieur, carbamoyl-alkyle inférieur N- mono- ou N,N-disubstitué, cycloalkyle, (alcoxy inférieur)carbonyle, carboxy, phényle, phényle substitué, pyridinyle, pyrimidinyle, ou pyrazinyle ;
R₄ représente l'hydrogène, un groupe alkyle inférieur ou un halogène ; et
le préfixe « inférieur » représente un radical comportant jusqu'à 7 atomes de carbone, 7 inclus, les radicaux en question étant linéaires ou ramifiés avec une ramification unique ou des ramifications multiples,
pour fabriquer une composition pharmaceutique destinée au traitement d'une maladie qui dépend de l'activité d'une protéine kinase, ladite maladie étant l'hypertension pulmonaire.

2. Utilisation d'un pyrimidylaminobenzamide de formule I selon la revendication 1, ou d'un N-oxyde ou sel de celui-ci acceptable sur le plan pharmaceutique, pour fabriquer une composition pharmaceutique destinée au traitement d'une maladie qui dépend de l'activité de la Tie-2 kinase, ladite maladie étant l'hypertension pulmonaire.

3. Utilisation selon l'une quelconque des revendications 1 ou 2, dans laquelle le composé de formule I est le 4-méthyl-3-[[4-(3-pyridinyl)-2-pyrimidinyl]amino]-N-[5-(4-méthyl-1H-imidazol-1-yl)-3-(trifluorométhyl)phényl]-benzamide.

4. Pyrimidylaminobenzamide de formule I, ou N-oxyde ou sel de celui-ci acceptable sur le plan pharmaceutique, dans lequel le pyrimidylaminobenzamide de formule I présente la structure suivante : 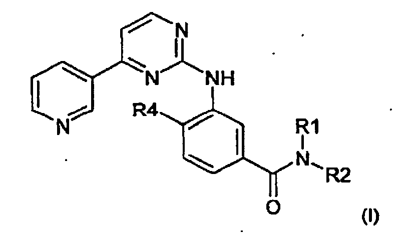 dans laquelle
R₁ représente l'hydrogène, un groupe alkyle inférieur, alcoxy inférieur-alkyle inférieur, acyloxy-alkyle inférieur, carboxy-alkyle inférieur, (alcoxy inférieur)carbonyl-alkyle inférieur ou phényl- alkyle inférieur ;
R₂ représente l'hydrogène, un groupe alkyle inférieur, éventuellement substitué par un ou plusieurs radicaux R₃ identiques ou différents, cycloalkyle, benzocycloalkyle, hétérocyclyle, un groupe aryle, ou un groupe hétéroaryle mono- ou bicyclique comprenant zéro, un, deux ou trois atomes d'azote cyclique et zéro ou un atome d'oxygène et zéro ou un atome de soufre, lesdits groupes étant dans chaque cas non substitués ou mono- ou polysubstitués ; et
R₃ représente un groupe hydroxy, alcoxy inférieur, acyloxy, carboxy, (alcoxy inférieur)carbonyle, carbamoyle, carbamoyle N- monosubstitué ou N,N-disubstitué, amino, amino mono- ou disubstitué, cycloalkyle, hétérocyclyle, un groupe aryle, ou un groupe hétéroaryle mono- ou bicyclique comprenant zéro, un, deux ou trois atomes d'azote cyclique et zéro ou un atome d'oxygène et zéro ou un atome de soufre, lesdits groupes étant dans chaque cas non substitués ou mono- ou polysubstitués ; ou dans laquelle
R₁ et R₂ conjointement représentent un groupe alkylène comportant quatre, cinq ou six atomes de carbone, éventuellement mono- ou disubstitué par un groupe alkyle inférieur, cycloalkyle, hétérocyclyle, phényle, hydroxy, alcoxy inférieur, amino, amino mono- ou disubstitué, oxo, pyridyle, pyrazinyle ou pyrimidinyle ; benzalkylène comportant quatre ou cinq atomes de carbone ; oxoalkylène comportant un oxygène et trois ou quatre atomes de carbone ; ou azaalkylène comportant un atome d'azote et trois ou quatre atomes de carbone, où l'atome d'azote est non substitué ou substitué par un groupe alkyle inférieur, phényl-alkyle inférieur, (alcoxy inférieur)carbonyl-alkyle inférieur, carboxy-alkyle inférieur, carbamoyl-alkyle inférieur, carbamoyl-alkyle inférieur N- mono- ou N,N-disubstitué, cycloalkyle, (alcoxy inférieur)carbonyle, carboxy, phényle, phényle substitué, pyridinyle, pyrimidinyle, ou pyrazinyle ;
R₄ représente l'hydrogène, un groupe alkyle inférieur ou un halogène ; et
le préfixe « inférieur » représente un radical comportant jusqu'à 7 atomes de carbone, 7 inclus, les radicaux en question étant linéaires ou ramifiés avec une ramification unique ou des ramifications multiples,
destiné à une utilisation dans le traitement d'une maladie qui dépend de l'activité d'une protéine kinase, ladite maladie étant l'hypertension pulmonaire.

5. Pyrimidylaminobenzamide de formule I selon la revendication 4, ou N-oxyde ou sel de celui-ci acceptable sur le plan pharmaceutique, à utiliser dans le traitement d'une maladie qui dépend de l'activité de la Tie-2 kinase, ladite maladie étant l'hypertension pulmonaire.

6. Pyrimidylaminobenzamide de formule I selon la revendication 4 ou 5, ou N-oxyde ou sel de celui-ci acceptable sur le plan pharmaceutique, à utiliser dans le traitement d'une maladie qui dépend de l'activité de la Tie-2 kinase, ladite maladie étant l'hypertension pulmonaire, où le composé de formule I est le 4-méthyl-3-[[4-(3-pyridinyl)-2-pyrimidinyl]amino]-N-[5-(4-méthyl-1H-imidazol-1-yl)-3-(trifluorométhyl)phénylJ-benzamide.
